**Europäisches Patentamt**

**European Patent Office**

**Office européen des brevets**

(11) Numéro de publication: **0 003 922**
**A1**

(12) **DEMANDE DE BREVET EUROPEEN**

(21) Numéro de dépôt: **79400058.8**

(22) Date de dépôt: **29.01.79**

(51) Int. Cl.²: **C 07 C 49/48**
**C 07 C 45/24**

(30) Priorité: **07.02.78 FR 7803352**
**08.12.78 FR 7834579**

(43) Date de publication de la demande:
**05.09.79 Bulletin 79/18**

(84) Etats contractants désignés:
**BE CH DE FR GB LU NL SE**

(71) Demandeur: **PRODUITS CHIMIQUES UGINE KUHLMANN**
**Service Propriété Industrielle Tour Manhattan**
**F-92087 PARIS LA DEFENSE 2 Cédex 21 (FR)**

(72) Inventeur: **Cheminal, Bernard**
**"Le Clos Grillet"**
**F-73130 La Chambre(FR)**

(72) Inventeur: **Kiener, Paul**
**15, Chemin de la Citadelle**
**F-69230 St. Genis Laval(FR)**

(74) Mandataire: **Monceaux, Pierre et al,**
**PRODUITS CHIMIQUES UGINE KUHLMANN Service**
**Propriété Industrielle**
**Tour Manhattan Cédex 21 F-92087 Paris La Defense(FR)**

(54) **Procédé d'obtention d'isophorone incolore et stable.**

㊗ Procédé d'obtention d'isophorone incolore stable consistant à distiller le mélange brut de synthèse en vue d'éliminer la soude, l'acétone et la majeure partie de l'eau, puis à effectuer sur le mélange ainsi obtenu, à chaud, un traitement par une résine échangeuse d'ions acide fort sous forme acide, éventuellement séchée, à neutraliser l'effluent du réacteur contenant la résine échangeuse d'ions par un agent alcalin, à tamponner le mélange et le laver à l'eau par décantation avant de le distiller sous pression réduite.

EP 0 003 922 A1

- 1 -

## Procédé d'obtention d'isophorone incolore et stable

La présente invention a pour but de décrire un procédé d'obtention d'isophorone incolore et non-acide par traitement d'isophorone colorée au moyen de résines échangeuses d'ions, suivi d'une neutralisation de l'acidité éventuellement présente, et d'une rectification de l'isophorone après extraction par l'eau des sels ainsi formés.

Il est connu que l'isophorone peut être préparée par condensation alcaline de l'acétone, à température élevée et sous pression. Mais à côté de l'isophorone, se forment des produits secondaires, dont certains, tels l'oxyde de mésityle, peuvent être facilement éliminés par distillation, alors que d'autres ont des points d'ébullition tels qu'ils sont difficilement séparables de l'isophorone. Il en est ainsi en particulier pour des composés qui, bien que légèrement plus volatils que l'isophorone, ne le sont pas assez pour être séparés par distillation, même en utilisant des colonnes à distiller à haut pouvoir séparateur.

Ces composés sont fortement colorés en jaune et ne permettent donc pas l'obtention d'isophorone commerciale incolore. En outre, leur présence accélère l'élévation progressive de la coloration de l'isophorone et l'augmentation de son acidité lors de son stockage.

La présence de ces produits dans l'isophorone industrielle

- 2 -

est donc particulièrement indésirable et nécessite la mise au point d'un procédé permettant leur élimination et conduisant ainsi à un produit limpide et incolore, conservant ces propriétés au stockage.

Le brevet français 1.205.799 propose de traiter l'isophorone colorée sèche par un acide sulfonique aromatique à une température comprise entre 80°C et 160°C et plus particulièrement à une température voisine de 130°C, puis de séparer ensuite par distillation l'isophorone du mélange résultant.

Cependant, l'acide sulfonique introduit dans l'isophorone à traiter est éliminé avec les résidus de distillation, ce qui rend le procédé peu économique. En outre, si l'isophorone obtenue est bien incolore, l'acide mis en oeuvre provoque la dégradation d'une partie de cette dernière, abaissant ainsi le rendement de façon notable. Enfin, des phénomènes de corrosion inévitables se produisent dans le cas où le matériel utilisé est en acier, et si l'on peut pallier ce dernier inconvénient en opérant avec du matériel construit en matériaux adaptés, l'opération est rendue très onéreuse.

La brevet français 1.215.326 permet d'obtenir de l'isophorone incolore en traitant l'isophorone colorée par de la terre de foulon. Cette dernière est un produit naturel appartenant au groupe des silicates d'aluminium connus sous le nom de mont-morillonite. La qualité utilisée dans le procédé décrit est telle que le pH de sa suspension dans de l'eau distillée soit inférieur à 7.

Comme pour tout produit naturel les caractéristiques des terres de foulon sont sujettes à variation et il n'est pas opportun de baser la préparation d'un produit industriel comme l'isophorone sur ce genre d'auxiliaire de fabrication.

La demanderesse a mis au point un procédé qui remédie à ces différents inconvénients et permet d'obtenir de façon particulièrement simple, au moyen de produits industriels parfaitement définis, de l'isophorone incolore et peu acide conservant ses propriétés lors d'une longue période de stockage.

Ce procédé consiste à mettre en contact le mélange contenant de l'isophorone que l'on désire purifier avec des résines échangeuses d'ions acide fort sous forme acide ($H^+$) à une température suffisante pour permettre la transformation des impuretés colorées responsables de la mauvaise qualité de l'isophorone industrielle en composés non volatils puis à isoler par distillation l'isophorone incolore du mélange réactionnel obtenu.

Le mélange contenant l'isophorone à purifier peut être le mélange réactionnel issu d'un réacteur de condensation de l'acétone dont on aura éliminé au préalable l'acétone non transformée, la plus grande part de l'eau de réaction et l'agent alcalin, catalyseur de la réaction de condensation, mélange désigné sous le terme d'isophorone brute.

Il est également possible de mettre en oeuvre ce mélange après en avoir éliminé les composés moins volatils que l'isophorone en particulier ceux connus sous le nom de xylithones. Le mélange ainsi traité est désigné par le terme d'isophorone (brute) équeutée. Il contient encore un peu d'eau dissoute.

Néanmoins, il est plus intéressant de traiter un mélange dont auront été éliminés au maximum tous les composés pouvant être séparés de l'isophorone par distillation. Ces composés peuvent être en effet utilement recyclés dans le réacteur de catalyse alors qu'ils risqueraient d'être dégradés en composés inutilisables lors du passage sur la résine échangeuse d'ions. De plus, leur élimination réduit, pour une production donnée d'isophorone, le volume du mélange à traiter. Elle permet également, en augmentant la concentration des impuretés responsables de la coloration de l'isophorone, une meilleure transformation de ces dernières. Un tel mélange coloré contenant de l'isophorone à isoler, obtenu par élimination des composés moins volatils, et nettement plus volatils que l'isophorone, est appelé isophorone (brute) équeutée et étêtée. Une certaine quantité d'eau, de l'ordre de 1 %, peut encore être présente dans ce mélange.

Toutefois, le contact entre les produits à traiter et les sites acides de la résine pouvant être gêné par une quantité d'eau trop importante, il est plus avantageux de ne mettre en oeuvre que des mélanges ne contenant que des quantités d'eau nettement inférieures à 1 %, de l'ordre de 0,1 % à 0,3 %. Ces mélanges relativement anhydres peuvent être obtenus par distillation hétéroazéotropique avec élimination de l'eau en se servant comme agent d'azéotropisme des constituants du mélange même. Ces mélanges ainsi séchés sont appelés isophorone (brute) équeutée, étêtée et séchée.

Les résines échangeuses d'ions permettent de transformer les composés de volatilité voisine de celle de l'isophorone et responsable de la coloration indésirable du produit industriel en composés moins volatils dont la séparation d'avec l'isophorone est plus aisée par la suite. Ces résines échangeuses d'ions sont des résines échangeuses d'ions acide fort sous la forme acide, dite encore H$^+$.

Ces résines sont des composés organiques macromoléculaires, réticulés, dont certaines molécules des monomères constitutifs portent des substituants à caractère acide fort, tel par exemple le groupement $-SO_3H$. Les polymères support de ces groupements sont des copolymères phénol-formol ou styrène-divinylbenzène.

Un certain nombre de ces résines échangeuses d'ions, du fait de leur mode de préparation, sont dites "macroporeuses" et c'est de préférence ce type de résine qui est mis en oeuvre dans le cadre de l'invention. D'une manière générale toutes les résines échangeuses d'ions sont commercialisées sous des noms de marque et si la présente invention a été mise au point avec une résine vendue sous le nom d'"AMBERLYST 15", marque déposée de la Société ROHM AND HAAS FRANCE, il est bien entendu qu'elle n'est pas limitée à cette marque de résine, choisie seulement pour des raisons d'approvisionnement, et que toute marque ayant des caractéristiques semblables est utilisable.

Ces résines se présentent sous forme de petites billes dont le diamètre apparent est compris entre 0,3-0,4 mm et 1,2-1,5 mm le diamètre apparent moyen étant de l'ordre de 0,5 à 0,7 mm. Elles possèdent du fait de leur procédé de fabrication une porosité importante, de l'ordre de 25 à 45 % due à des macropores de diamètre 100 $\overset{o}{A}$ et plus et une surface spécifique très supérieure à 10 m2/g pouvant aller jusqu'à 50 m2/g. Enfin leur capacité d'échange, qui s'exprime en milliéquivalents/g, est comprise entre 4 et 5. Toutes ces valeurs sont données pour des résines à l'état sec.

Ainsi la résine AMBERLYST 15 est constituée de sphéroïdes à l'état sec dont la répartition granulométrique est la suivante :

- taille supérieure à 1,2 mm : 2,4 %
- taille comprise entre 1,2 mm et 0,84 mm : 24,2 %
-     "        "         "     0,84 mm et 0,6 mm : 47,9 %
-     "        "         "     0,6 mm et  0,4 mm : 18,8 %
-     "        "         "     0,4 mm et  0,3 mm : 5,7 %
- taille inférieure à 0,3 mm : 1 % au maximum.

Sa porosité est de 30 à 35 %, sa surface spécifique de 40 à 50 m2/g avec des macropores de diamètre moyen compris entre 200 et 600 Å. La capacité d'échange de cette résine est de 4,9 milliéquivalents.

Certaines résines échangeuses d'ions sont livrées à l'état sec et peuvent être mises en oeuvre selon la présente invention sans traitement préalable. Il en est ainsi de la résine "AMBERLYST 15". D'autres, en revanche, sont livrées saturées d'eau. Il est nécessaire dans ce cas avant de les mettre en oeuvre, de procéder à leur séchage selon les indications des fabricants. Une méthode particulièrement adaptée à la présente invention est d'opérer une distillation hétéroazéotropique en utilisant de l'isophorone comme agent d'azéotropisme.

La température à laquelle s'effectue le traitement de l'isophorone doit être suffisante pour que les impuretés soient transformées. Une température minimale de 80°C est nécessaire. Par ailleurs, la température maximale à laquelle est effectuée la réaction ne peut dépasser la température à partir de laquelle les résines échangeuses d'ions commencent à perdre leur stabilité, température voisine de 120°C. Le domaine utile de la température de réaction compris entre ces valeurs varie de 100 à 115°C.

Le procédé de l'invention peut être mis en oeuvre de plusieurs façons. L'isophorone est, par exemple, traitée selon un mode discontinu, une certaine quantité d'isophorone réagissant pendant un temps donné avec une quantité déterminée de résine, soit dans un réacteur agité classique, soit en faisant circuler plusieurs fois de suite l'isophorone à traiter sur un lit de résine.

Il est également possible d'opérer de façon continue en faisant traverser une seule fois par l'isophorone à traiter le lit de résine échangeuse d'ions, avec un temps de séjour tel que les impuretés colorées soient transformées. Dans ce cas le temps de séjour, défini par le rapport $\dfrac{\text{volume de résine}}{\text{débit d'isophorone}}$ , doit être compris entre 30 mn et 4 h et de préférence entre 1 h et 2 h.

Ce traitement de décoloration peut être également combiné avec l'opération d'élimination des sous-produits facilement séparables de l'isophorone ainsi qu'avec l'opération d'isolement de l'isophorone incolore, opérations qui s'effectuent par distillation sous pression réduite selon des techniques connues.

Cependant, l'effluent du réacteur de décoloration contient une ou des substances légèrement plus volatiles que l'isophorone et présentant une certaine acidité. Cette ou ces substances se retrouvent, si certaines précautions ne sont pas prises, dans l'isophorone décolorée lors de l'isolement de cette dernière.

La présence inopportune de cette acidité nuit à la bonne conservation de l'isophorone décolorée et la rend donc rapidement non commercialisable car non conforme aux spécifications en vigueur.

La demanderesse a constaté également que l'acidité présente dans l'effluent du réacteur diminuait au fur et è mesure du vieillissement de la résine, ce qui entrainait corrélativement une décroissance de l'acidité de l'isophorone obtenue, mais cet abaissement de l'acidité n'est pas suffisamment important ni rapide pour que l'isophorone distillée directement soit de bonne qualité. Parmi les précautions qui peuvent être prises pour obtenir un produit commercialisable et stable, on peut citer par exemple et à titre non limitatif, le prélèvement d'une fraction de tête très acide si l'effluent du réacteur de décoloration est soumis à une rectification fractionnée ou encore, par un prélèvement latéral de l'isophorone commerciale et le coulage en tête de colonne d'une fraction acide recyclable dans la
réaction de synthèse de façon continue.

**Néanmoins** ces mesures ne sont pas entièrement satisfaisantes
car elles provoquent une perte de productivité de l'installation de production d'isophorone du fait des recyclages
qu'elles exigent et elles sont coûteuses en énergie, sous
forme de vapeur, puisqu'elles compliquent les distillations.

Il est possible de pallier ces inconvénients en effectuant
sur l'effluent brut du réacteur à résine une extraction
des substances acides qu'il contient par une série de lavages avec un solvant non miscible à cet effluent et où en
revanche les substances acides se dissolvent facilement.
Parmi les composés utilisables l'eau est le solvant préféré.

Toutefois ce procédé n'est pas tout à fait valable sur le
plan pratique, car il exige un nombre d'étages d'extraction
élevé et donne lieu à une perte non négligeable d'isophorone
dans l'eau utilisée étant donné l'importance du volume d'eau
mis en oeuvre par rapport à celui d'effluent traité. Par
ailleurs l'acidité de l'isophorone pure obtenue finalement,
exprimée en acide acétique, et avant tout vieillissement
est de l'ordre de 0,004 % ce qui est relativement important.

Un traitement de l'effluent du réacteur à résine supprimant
ces derniers inconvénients a été finalement mis au point
par la demanderesse. Il consiste à neutraliser l'acidité
présente dans l'isophorone, à extraire les sels ainsi formés par lavage à l'eau et à isoler l'isophorone commerciale
par rectification.

La neutralisation de l'acidité présente dans l'effluent du
réacteur de décoloration doit être complète. Aussi un léger
excès d'agent neutralisant doit être employé. Un excès de
15 % de la quantité d'agent neutralisant stoechiométrique- ment nécessaire est convenable.

Dans la pratique, cette neutralisation est effectuée sous agitation, au moyen d'une solution aqueuse d'hydroxyde de sodium. Pour que cette neutralisation soit efficace, il est nécessaire que le mélange neutralisé, principalement composé d'isophorone et d'une solution aqueuse de l'hydroxyde de sodium en excès et des sels formés reste homogène. Ceci oblige à limiter la quantité d'eau à introduire à la teneur qui peut être dissoute par l'isophorone, soit par exemple 3,5 % à 80°C. La concentration en soude de cette solution doit être suffisante pour neutraliser la totalité de l'acidité présente, compte tenu du volume qui est mis en oeuvre. Pratiquement cette concentration est comprise entre 10 et 30 g/1 et est habituellement de l'ordre de 20 g/1.

La température à laquelle s'effectue cette neutralisation n'est pas critique et il suffit de refroidir légèrement l'effluent du réacteur, par exemple à 80°C, avant d'introduire la solution aqueuse d'hydroxyde de sodium. Mais il est possible également d'opérer à température plus basse.

Une distillation directe du mélange ainsi alcalinisé risquerait de provoquer des phénomènes de polymérisation de l'isophorone. Aussi l'excès de soude est neutralisé à son tour par addition sous agitation d'une solution aqueuse d'acide phosphorique à 10 g/1 de façon à ce que le pH d'un mélange obtenu par addition de 25 ml d'un échantillon du milieu réactionnel final dans 100 ml d'une solution hydro-méthanolique à 50 % en volume soit compris entre 6,5 et 7. La température à laquelle cette addition d'acide est faite n'est pas critique. Elle peut être comprise entre 80°C et la température ambiante.

Au cours de ces diverses neutralisations il se forme des sels de sodium qui doivent être éliminés du milieu. Ceci est commodément réalisé par addition d'eau. L'addition d'eau doit être suffisante pour permettre la dissolution des sels présents mais elle ne doit pas être trop importante car l'eau dissout 2 % de son poids d'isophorone et cette dernière doit être récupérée. Le rapport $\dfrac{\text{volume d'eau}}{\text{volume produit organique}}$ doit donc être compris entre 0,2 et 1 et pratiquement est voisin de 0,25.

La dissolution des sels est facilitée par l'agitation du mélange qui est ensuite décanté. La phase aqueuse est tout d'abord envoyée dans une colonne de stripping pour récupérer l'isophorone dissoute alors que la couche organique est distillée.

Cette distillation permet tout d'abord d'éliminer l'eau par hétéroazéotropie par distillation continue en tête de colonne sans étêtage préalable, puis d'obtenir ensuite l'isophorone commerciale incolore et non acide.

Les exemples suivants sont donnés à titre d'illustration de l'invention et afin de mettre en évidence les modalités d'exécution de celle-ci, mais ne sont aucunement limitatifs de la mise en oeuvre de cette dernière. La coloration de l'isophorone est mesurée dans un colorimètre LOVIBOND 100 et exprimée en unités Hazen. (Echelle platine-cobalt selon la norme ASTM D 1209.62).

EXEMPLE 1.

Dans un réacteur en verre de 2 litres équipé avec un agitateur et un réfrigérant ascendant, contenant 150 ml de résine échangeuse d'ions AMBERLYST 15 sous forme $H^+$, lavée trois fois avec du méthanol puis une fois avec de l'isophorone à décolorer, on introduit 1 000 ml d'isophorone équeutée et séchée par distillation azéotropique. Ce mélange contient donc des composés plus volatils que l'isophorone en particulier ceux fortement colorés en jaune.

Ce mélange est porté à 102°C. On constate très rapidement un noircissement de la masse réactionnelle, caractéristique de la transformation des impuretés colorées.

Au bout de 7 heures de réaction le mélange réactionnel est séparé de la résine puis distillé sous pression réduite.

Une fraction de tête de 100 ml est d'abord prélevée. Elle contient 80 % d'isophorone et sa coloration est de 8 unités Hazen. Une seconde fraction de 700 ml est ensuite prélevée. Sa coloration est comprise entre 5 et 8 unités Hazen. La température en tête de colonne s'élève alors et la fraction obtenue ensuite est fortement colorée en jaune par des composés moins volatils que l'isophorone. Une distillation témoin effectuée sur l'isophorone de départ sans traitement préalable permet de recueillir une fraction de coeur ayant une coloration de 65 unités Hazen.

EXEMPLE 2.

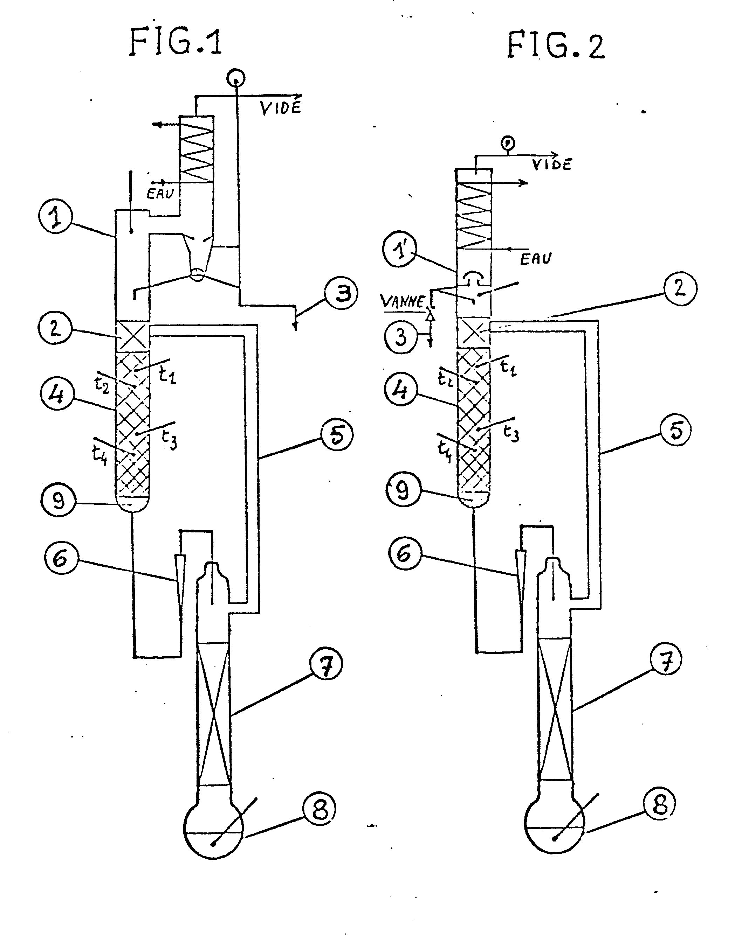

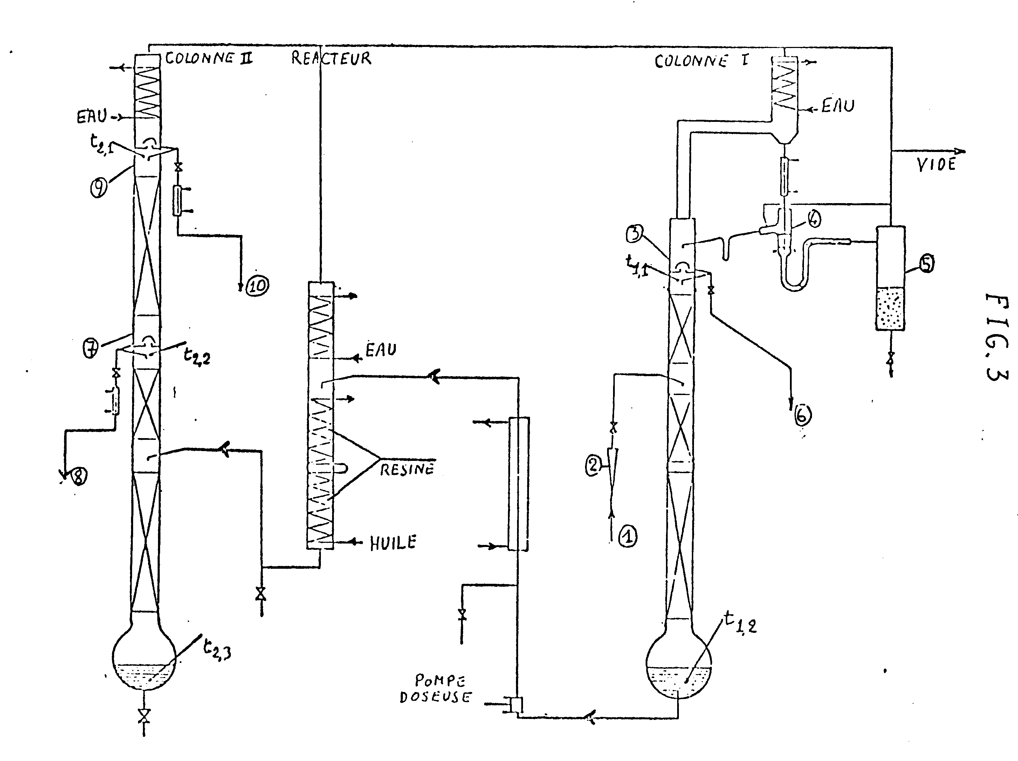

On opère dans l'appareil représenté sur la figure 1 de la planche 1/2 et construit avec du matériel en verre borosilicaté, tel par exemple celui commercialisé par la Société EIVS.

Cet appareil est constitué par un ballon à distiller (6) de 5 l. de capacité surmonté d'une colonne à distiller (7) longue de 1 m, de diamètre nominal 50 mm et remplie de 6 tampons en fil d'acier inoxydable tricoté MULTIKNIT, marque déposée de la Société TISSMETAL. Ces garnitures MULTIKNIT ont un diamètre de 50 mm et une longueur de 15 cm.

Au sommet de cette colonne une tubulure (5) conduit les vapeurs du mélange mis en distillation dans un tampon en fil d'acier tricoté MULTIKNIT (2) puis dans une tête de colonne (1) elle-même surmontée d'un condenseur refroidi à l'eau et au travers duquel on peut ajuster la pression interne dans l'appareil au moyen d'une installation de vide.

La tête de colonne, à commande manuelle, permet de réaliser la décantation de mélanges hétérogènes et le prélèvement de la phase inférieure de tels mélanges en renvoyant la phase supérieure sur le tampon (2). Elle permet aussi, si le distillat est homogène, d'en prélever éventuellement un échantillon par la tubulure (3), sans modifier sensiblement le régime de l'installation.

Le liquide condensé après passage dans la tête de colonne (1) et le tampon (2) s'écoule au travers d'un lit d'une résine échangeuse d'ions placée dans un élément de diamètre 50 mm et longueur 75 cm (4), équipé de quatre thermomètres $t_1$ à $t_4$.

Un remplissage (9) et le tampon (2) maintiennent en place les grains de la résine. Le liquide ayant traversé le lit de résine retourne au travers d'un débitmètre (6) en tête de la colonne (7).

1 400 ml de cette résine AMBERLYST 15 lavée à l'isophorone brute équeutée sont placés dans l'élément (4) et 4,2 kg d'isophorone brute équeutée sont introduits dans le ballon (8).

Dans un premier temps le condensat obtenu en (1) est hétérogène et l'eau décantée est éliminée par (3). L'appareil est alors mis en régime et fonctionne sous une pression de 50 mm de mercure, le débit du distillat mesuré en (6) étant de 3,5 l/h.

On note les températures suivantes au bout de 7 heures de marche :

$$\text{Températures} = \text{tête de colonne (1)} = 123$$
$$\text{(en °C)} \quad t_1 = 111$$
$$t_2 = 111$$
$$t_3 = 91$$
$$t_4 = 90$$
$$\text{ballon (8)} = 131$$

L'appareil est alors arrêté et le mélange contenu dans le ballon (8) mis à distiller. Il fournit 3 700 g d'isophorone ayant une coloration de 10 unités Hazen, et 200 g d'isophorone mélangée à des produits lourds, fraction recyclable en distillation.

Une distillation effectuée à titre de témoin sur le mélange initial n'ayant pas subi le traitement de l'invention fournit sensiblement la même quantité d'isophorone mais sa coloration est de 65 unités Hazen.

EXEMPLES 3 à 6.

L'appareil utilisé dans l'exemple 2 est légèrement modifié, la tête de colonne (1) étant remplacée par une tête (1') (voir fig. 2 planche 1/2) qui ne permet plus de réaliser la décantation de mélanges hétérogènes mais qui présente l'avantage d'avoir un contrôle électronique du taux de reflux, la vanne équipant la tubulure (3) étant à commande électromagnétique pilotée par un appareil de contrôle du temps de marche.

Pour réaliser les différents exemples, le ballon (8) est chargé avec 4,2 kg d'isophorone brute équeutée, séchée par distillation azéotropique et partiellement étêtée.

Le chauffage du ballon étant branché et l'appareil étant mis sous pression réduite, de l'ordre de 50 mm de mercure, une circulation d'isophorone semblable à celle décrite dans l'exemple ci-dessus s'établit.

- 14 -

Le débit de liquide passé sur la résine, mesuré en 6, est fonction de la puissance fournie par le système de chauffage du ballon.

A des intervalles de temps réguliers un petit échantillon du distillat est prélevé par la tubulure (3) dans la tête de colonne (1') le taux de reflux étant alors fixé à 10/1, et sa coloration est mesurée.

Le tableau ci-dessous indique en unités Hazen la coloration de divers échantillons prélevés au cours du temps lors d'essais réalisés avec divers débits de liquide. Le temps O est pris quand les premières vapeurs arrivent dans la tête de colonne (1').

| Durée de la réaction (en h) | Débits de liquide (en 1/h) | | | |
|---|---|---|---|---|
| | 1,5 | 3,3 | 5,3 | 9 |
| 1 | | 35 | 35 | 35 |
| 2 | | 12 | 10 | 20 |
| 3 | 30 | 8 | 8 | 17 |
| 4 | | 5 | 5 | 12 |
| 5 | 18 | < 5 | < 5 | non mesuré |
| 6 | | | | |
| 7 | 8 | | | |

Les températures enregistrées après 5 h de réaction dans les essais réalisés avec les débits de 3,3 et 5,3 1/h étaient respectivement les suivantes :

| | Températures en °C | |
| --- | --- | --- |
| | Essai à 3,3 l/h | Essai à 5,3 l/h |
| Tête de colonne (1') | 121 | 118 |
| $t_1$ | 114 | 113 |
| $t_2$ | 112 | 112 |
| $t_3$ | 93 | 95 |
| $t_4$ | 92 | 95 |
| Ballon (8) | 132 | 127 |

La coloration initiale de l'isophorone distillée et prélevée en (3) était de l'ordre de 60 unités Hazen.

Il apparaît donc que le traitement selon l'invention est particulièrement efficace pour décolorer l'isophorone lorsque le produit à traiter est maintenu suffisamment longtemps en contact avec les résines échangeuses d'ions.

Par ailleurs la stabilité dans le temps de la coloration de l'isophorone obtenue a été vérifiée sur des échantillons prélevés en (3) après 5 h de réaction dans le cas de l'essai réalisé à 3,3 l/h et conservé dans des flacons en verre, à la lumière.

On enregistre les colorations suivantes en fonction du temps :

| Durée de conser- vation | Isophorone non traitée | Echantillon en flacon fermé | Echantillon en flacon ouvert |
|---|---|---|---|
| 0 jour | 60 | 5 | 5 |
| 7 jours | 80 | 5 | 10 |
| 33 jours | non mesurable | 12 | 45 |

EXEMPLE 7.

Cet exemple illustre le procédé de l'invention dans laquelle de l'isophorone brute équeutée est mise en oeuvre et obtenue incolore de façon entièrement continue.

L'essai est réalisé dans l'appareil représenté sur la planche 2/2 et qui comporte trois parties, soit de droite à gauche :

- une colonne à distiller en continu I recevant l'isophorone équeutée à traiter et qui a pour rôle de sécher et d'éliminer les composés nettement plus volatils que l'isophorone.

- un réacteur de traitement des impuretés colorées.

- une colonne à distiller en continu II recevant l'effluent du réacteur précédent et délivrant de l'isophorone pure incolore.

D'une manière plus précise, l'introduction de l'isophorone équeutée à traiter qui contient environ 3 % en poids d'eau, se fait dans la colonne I par la tubulure latérale (1) au travers d'un débitmètre (2).

La colonne I est constituée par un ballon de 10 l (bouilleur à niveau constant), 3 viroles de diamètre intérieur 50 mm et de longueur respective 1 m, 0,5 m et 0,5 m, remplies respectivement de 6,2 et 2 tampons MULTIKNIT, une tête de colonne (3) identique à celle utilisée dans les exemples 3 à 6, un condenseur de vapeur montante, un décanteur des vapeurs condensées (4) permettant le retour dans la colonne, au dessus de la tête (3), de la phase organique supérieure et l'accumulation dans une capacité (5) de la couche aqueuse inférieure. La tubulure (6) qui équipe la tête (3) permet de recueillir à l'extérieur de la colonne une fraction de la phase organique provenant du décanteur (4) et qui est constituée d'isophorone contenant des composés plus volatils qu'elle et qu'il est possible de recycler dans le réacteur de synthèse.

Des thermomètres $t_{1,1}$ et $t_{1,2}$ donnent les températures respectives en tête et en pied de colonne.

L'isophorone brute équeutée, étêtée et séchée est reprise dans le bouilleur de la colonne I par une pompe doseuse et injectée après passage dans un échangeur-réchauffeur dans la partie supérieure du réacteur à résine échangeuse d'ions qui est surmonté d'un échangeur-refroidisseur destiné à condenser et à rétrograder les vapeurs d'isophorone à traiter qui auraient pu se dégager.

Le réacteur utilisé ici est une capacité en verre traversée par un serpentin.

La résine échangeuse d'ions (1,85 l d'AMBERLYST 15 sous forme $H^+$) est placée à l'intérieur de ce réacteur et maintenue à la température désirée pour la réaction par une circulation d'huile caloporteuse thermostatée à travers le serpentin. Elle est entièrement immergée dans le mélange en cours de traitement.

L'effluent du réacteur alimente la colonne à distiller II de diamètre 50 mm, qui est constituée par un ballon de 10 l (bouilleur), 2 viroles de longueur 1 m et 0,5 m contenant respectivement 6 et 2 tampons MULTIKNIT, séparées par l'introduction de l'effluent du réacteur et surmontées d'un système de prélèvement latéral (7) permettant l'écoulement d'une fraction du liquide en reflux par la tubulure (8) (isophorone commerciale). L'appareil est encore surmonté d'une virole de 1 m (6 tampons MULTIKNIT), d'une tête de colonne (9) équipée d'un condenseur de vapeur et d'un coulage d'une fraction des vapeurs condensées en tête par la tubulure (10) (isophorone acide recyclée dans le réacteur de synthèse). Les produits lourds résultant de l'action de la résine échangeuse d'ions s'accumulent dans le bouilleur d'où ils sont périodiquement éliminés.

Des thermomètres $t_{2,1}$, $t_{2,2}$, $t_{2,3}$ donnent les températures respectives en tête de colonne, dans le système de prélèvement latéral (7) et en pied de colonne.

Enfin l'ensemble des colonnes et du réacteur est mis sous pression réduite au moyen d'un collecteur branché sur une installation de vide.

Dans cette installation on applique les conditions opératoires suivantes :

| | |
|---|---|
| Pression | = 45 mm de mercure |
| Débit en (2) | = 1,25 l/h d'isophorone brute équeutée |
| $t_{1,1}$ | = 80 à 90°C |
| $t_{1,2}$ | = 129°C |
| Débit en (5) | = 35 ml/h |
| Débit en (6) | = 25 ml/h |
| Température de réaction | = 111°C |
| $t_{2,1}$ | = 118 - 119°C |
| $t_{2,2}$ | = 121,5°C |
| $t_{2,3}$ | = 135°C |

Taux de reflux en (9)   =   24/1
Débit en (10)           =   70 ml/h
Taux de reflux en (7)   =    4/1
Débit en (8)            = 1 100 ml/h

L'installation étant en fonctionnement stable on prend des échantillons aux tubulures (8) et (10).

On note leur coloration et leur acidité en fonction du temps, en les conservant en flacon ouvert, à la lumière :

| Durée de Stockage | Isophorone prélevée en (8) | | Isophorone acide prélevée en (10) | |
|---|---|---|---|---|
| | Coloration | Acidité % | Coloration | Acidité % |
| 0 jour | 5 à 8 | 0,0024 | 5 à 8 | 0,05 |
| 15 jours | · 12 | 0,0072 | très fortement coloré (70) | 0,1 |
| 37 jours | 30 | 0,0096 | - | - |

Ainsi l'isophorone prélevée en 8 et conservée en flacon ouvert ne se colore que très lentement. Aucune mesure n'a été faite en flacon fermé car l'évolution est alors bien plus lente et aucune variation significative de la coloration n'aurait pu être observée. Un test comparatif a été effectué en distillant séparément sur une colonne à distiller efficace l'isophorone brute alimentant l'installation. Il n'a pas été possible d'obtenir de fractions ayant une coloration inférieure à 60.

Par ailleurs une distillation standard selon la norme ASTM D 1078 est effectuée sur un échantillon prélevé en (8).

On a les valeurs suivantes, en °C :

| | Norme ASTM | Produit commercial courant | Echantillon prélevé en (8) |
|---|---|---|---|
| Point initial | 205 | 209 | 210 |
| Point sec | 218 | 217 | 214 |

EXEMPLE 8.

Dans la même installation que celle décrite dans l'exemple 7 les conditions opératoires suivantes sont appliquées :

- pression = 44 mm de mercure
- débit en (2) = 1,87 l/h d'isophorone brute équeutée
- $t_{1,1}$ = 80 à 90°C
- $t_{1,2}$ = 127 à 128°C
- débit en (5) = 52 ml/h
- débit en (6) = 40 ml/h
- température de réaction = 111°C
- $t_{2,1}$ = 118°C
- $t_{2,2}$ = 121,5°C
- $t_{2,3}$ = 135°C
- taux de reflux en (9) = 32/1
- débit en (10) = 100 ml/h
- taux de reflux en (7) = 3/1
- débit en (8) = 1 640 ml/h

Des échantillons ont été prélevés aux tubulures (8) et (10) lorsque l'installation était en fonctionnement stable, et ils ont été conservés en flacon ouvert.

Leur coloration et leur acidité ont été régulièrement mesurées au cours du temps ce qui conduit aux résultats suivants :

| Durée du stockage | Isophorone prélevée en (8) | | Isophorone acide prélevée en (10) | |
|---|---|---|---|---|
| | Coloration | Acidité % | Coloration | Acidité % |
| 0 jour | 6 | 0,002 | 8 | 0,04 |
| 14 jours | 12 | 0,005 | très forte- ment colo- rée (70) | 0,09 |
| 36 jours | 30 | 0,01 | - | - |

Une distillation standard effectuée sur un échantillon prélevé en (8) donne les mêmes résultats que ceux observés à
l'exemple 7.

EXEMPLE 9.

Dans l'installation utilisée dans les exemples 7 et 8 on applique les conditions opératoires suivantes :

- pression       =   44   mm de mercure
- débit en (2)       =   2,3 l/h
- $t_{1,1}$       =   80 à 90°C
- $t_{1,2}$       = 128°C
- débit en (5)       =   70 ml/h
- débit en (6)       =   45 ml/h
- température de réaction       = 111°C
- $t_{2,1}$       = 120°C
- $t_{2,2}$       = 123°C
- $t_{2,3}$       = 138°C
- taux de reflux en (9)       = 2/1
- débit en (10)       = 390 ml/h

- taux de reflux en (7)     =   1/1
- débit en (8)              = 1 780 ml/h

Des échantillons prélevés comme précédemment sont analysés.
Voici les résultats :

| | Isophorone prélevée en (8) | Isophorone acide prélevée en (10) |
|---|---|---|
| Coloration | 8 | 10 |
| Acidité (%) | 0,004 | 0,086 |

Au stockage les échantillons prélevés en (8) ne se colorent
que très lentement.

EXEMPLE 10.

Un réacteur de décoloration constitué par un cylindre en
acier inoxydable thermostaté à 100°C contenant un demi-litre
de résine échangeuse d'ions AMBERLYST 15 I.G. soigneusement
séchée est alimenté sous la pression atmosphérique avec de
l'isophorone brute équeutée, étêtée et séchée au débit de
500 ml/h.

L'effluent de ce réacteur de couleur brun foncé a une acidité exprimée, en acide acétique, de 0,029 % (acidité mesurée par dosage avec de la potasse alcoolique 0,1 N en
présence de bleu de thymol sur un échantillon de 50 ml dilué dans de l'isophorone neutre et incolore).

4 litres de cet effluent maintenus à 80°C sont additionnés
de 44 ml d'une solution aqueuse d'hydroxyde de sodium à
20 g/l et conservés sous agitation pendant 15 minutes. Au
bout de ce temps, et toujours sous agitation, 9,5 ml d'une
solution aqueuse d'acide phosphorique à 10 g/l sont intro-

duits puis ensuite 1 litre d'eau bipermutée. Le tout est ensuite mis à décanter. Après séparation de la couche inférieure aqueuse, la couche organique est séchée par hétérodistillation puis introduite au milieu d'une colonne à distiller continue de diamètre 50 mm, à 16 plateaux théoriques, dont le régime de marche est le suivant :

- pression : 50 mm de mercure
- température tête : 119°C
- température pied : 126°C
- débit alimentation : 1 1/h
- débit coulage en tête : 940 ml/h
- taux de reflux : 2/1
- débit soutirage en pied : 60 ml/h

L'isophorone coulée en tête à la 4è heure de marche a les caractéristiques suivantes :

- coloration : 8
- acidité : 0,0018 %

EXEMPLE 11.

Un échantillon moyen de l'effluent du réacteur de décoloration tel qu'il fonctionne dans l'exemple 10, obtenu sur une période de marche d'une dizaine d'heures a une acidité de 0,06 %.

4 litres de cet échantillon sont additionnés, à 80°C, de 92 ml d'une solution aqueuse d'hydroxyde de sodium à 20 g/l sous agitation, maintenue pendant 15 minutes ; 20 ml d'une solution aqueuse d'acide phosphorique à 10 g/l sont ensuite versés dans ce mélange puis 1 litre d'eau bipermutée. Après décantation de la couche aqueuse et séchage par hétérodistillation, la couche organique est introduite dans la même colonne à distiller, fonctionnant avec le même régime de marche que dans l'exemple 10.

- 24 -

L'isophorone coulée en tête de colonne a les caractéristiques suivantes :

- coloration : 8
- acidité : 0,0015 %

Elle est conservée à la lumière en flacon de verr blanc, bouché, pendant 40 jours. Au bout de cette périod, elle a les caractéristiques suivantes :

- coloration : 15
- acidité : 0,0031 %

qui sont celles d'un produit commercialisable.

Revendications de brevet

1. Procédé d'obtention d'isophorone incolore stable consistant à distiller le mélange brut de synthèse en vue d'éliminer la soude, l'acétone et la majeure partie de l'eau, puis à effectuer sur le mélange ainsi obtenu, à chaud, un traitement par une résine échangeuse d'ions acide fort sous forme acide, éventuellement séchée, à neutraliser l'effluent du réacteur contenant la résine échangeuse d'ions par un agent alcalin, à tamponner le mélange et le laver à l'eau par décantation avant de le distiller sous pression réduite.

2. Procédé selon la revendication 1 où l'isophorone mise en oeuvre provient d'un mélange brut de synthèse étêté, équeuté et séché.

3. Procédé selon l'une des revendications 1 ou 2 où la teneur en eau est inférieure à 1 %, préférentiellement 0,3 %.

4. Procédé selon l'une des revendications 1 à 3 où le traitement est effectué à une température comprise entre 80 et 120°C, et de préférence entre 100 et 115°C.

5. Procédé selon l'une des revendications 1 à 4 où le temps de séjour du mélange brut de synthèse sur la résine échangeuse d'ions est compris entre 30 mn et 4 h, et de préférence entre 1 et 2 h.

6. Procédé selon l'une des revendications 1 à 5 où la résine échangeuse d'ions est de type macroporeux.

7. Procédé selon l'une des revendications 1 à 6 où la résine échangeuse d'ions est constituée de billes à l'état sec dont le diamètre apparent est compris entre 0,3 mm et 1,5 mm, et possèdent une porosité comprise entre 25 % et 45 %, une surface spécifique BET comprise entre 20 m2/g et

50 m2/g et des macropores de diamètres supérieurs à 100 Å.

8. Procédé selon l'une des revendications 1 à 7 fonctionnant en continu.

9. Procédé selon l'une des revendications 1 à 8 où le mélange brut de synthèse à traiter provient de la condensation alcaline de l'acétone à température élevée.

10. Procédé selon l'une des revendications 1 à 9 dans lequel la neutralisation de l'isophorone après traitement par la résine est effectuée avec une solution aqueuse d'hydroxyde de sodium dont la concentration est comprise entre 10 et 30 g/l de préférence voisine de 20 g/l, introduite avec un excès de 15 % par rapport à l'acidité à neutraliser, en quantité telle que le mélange reste homogène.

11. Procédé selon l'une des revendications 1 à 10 dans lequel le mélange neutralisé est tamponné par addition d'une solution aqueuse d'acide phosphorique à 10 g/l.

12. Procédé selon l'une des revendications 1 à 11 dans lequel le mélange neutralisé et tamponné est lavé par addition d'un volume d'eau égal au quart du volume du produit organique.

13. Procédé selon l'une des revendications 1 à 12 dans lequel l'isophorone incolore et stable est obtenue par distillation après séchage hétéroazéotropique de la couche organique.

FIG.1   FIG.2

COLONNE II    REACTEUR    COLONNE I

FIG. 3

2/2

0003922

## DOCUMENTS CONSIDERES COMME PERTINENTS

| Catégorie | Citation du document avec indication, en cas de besoin, des parties pertinentes | Revendication concernée |
|---|---|---|
| | FR - A - 2 050 731 (ESSO RESEARCH & ENGINEERING)<br>* Page 1, lignes 5-9; page 6, lignes 18-38; page 7, lignes 1-3 *<br><br>-- | |
| A | FR - A - 2 200 231 (BAYER)<br>* Revendications 1,7 *<br><br>---- | |

**CLASSEMENT DE LA DEMANDE (Int. Cl.²)**

C 07 C 49/48
45/24

**DOMAINES TECHNIQUES RECHERCHES (Int. Cl.²)**

C 07 C 49/48
45/24

**CATEGORIE DES DOCUMENTS CITES**

X: particulièrement pertinent
A: arrière-plan technologique
O: divulgation non-écrite
P: document intercalaire
T: théorie ou principe à la base de l'invention
E: demande faisant interférence
D: document cité dans la demande
L: document cité pour d'autres raisons

&: membre de la même famille, document correspondant

Le présent rapport de recherche a été établi pour toutes les revendications

| Lieu de la recherche | Date d'achèvement de la recherche | Examinateur |
|---|---|---|
| La Haye | 17-05-1979 | BONNEVALLE |

OEB Form 1503.1 06.78